# EUROPEAN PATENT SPECIFICATION

(11) **EP 2 895 171 B1**
(45) Date of publication and mention of the grant of the patent: **12.07.2017**
(21) Application number: 13773171.7
(22) Date of filing: 12.09.2013
(51) Int. Cl.: A61K 9/20, A61K 31/53

(54) **A STABLE PHARMACEUTICAL FORMULATION CONTAINING VARDENAFIL HYDROCHLORIDE**
STABILE PHARMAZEUTISCHE FORMULIERUNG MIT VARDENAFIL-HYDROCHLORID
FORMULATION PHARMACEUTIQUE STABLE CONTENANT DU CHLORHYDRATE DE VARDÉNAFIL

(30) Priority: 14.09.2012 CZ 20120637
(43) Date of publication of application: 22.07.2015
(73) Proprietor: Zentiva K.S., 102 37 Praha 10 (CZ)
(72) Inventor: LINEK, Jan, 149 00 Praha 4 (CZ); KOFRANKOVA, Monika, 149 00 Praha 4 (CZ); HANZLIK, Pavel, 110 00 Praha 1 (CZ); DAMMER, Ondrej, 253 01 Hostivice (CZ); TKADLECOVA, Marcela, 163 00 Praha 6 (CZ)
(74) Representative: Jirotkova, Ivana
(86) International application number: PCT/CZ2013/000104
(87) International publication number: WO 2014/040577

(56) References cited:
- WO-A1-2004/006894
- US-A1- 2010 159 003
- COOPER V BRETT ET AL: "Quantification of crystalline forms in active pharmaceutical ingredient and tablets by X-ray powder diffraction", JOURNAL OF PHARMACY AND PHARMACOLOGY, JOHN WILEY & SONS LTD, LONDON; GB, vol. 55, no. 9, 1 September 2003 (2003-09-01), pages 1323-1329, XP009111587, ISSN: 0022-3573, DOI: 10.1211/0022357021675

## Description

### Technical Field

The invention relates to a stable pharmaceutical formulation for oral administration of Vardenafil hydrochloride and a method of its preparation.

### Background Art

The pharmacologically active substance 2-[2ethoxy-5-(4ethyl-pipreazine-1-sulfonyl)-phenyl]-5-methyl-7-propyl-3H-imidazo[5,1f][1,2,3]triazin-4-one (also known under the generic name Vardenafil), Vardenafil hydrochloride and Vardenafil hydrochloride trihydrate are described in the international patent application WO99/24433A (BAYER, DE). The above mentioned compounds are inhibitors of c-GMP metabolizing phosphodiesterases (PDE I, PDE II a PDE V) and are useful for treatment for cardiovascular and cerebrovascular diseases and/or diseases of the genitourinary tract and for treatment of the erectile dysfunction. The commercially available composition LEVITRA® was developed in the form of a coated tablet with immediate release of the drug in three strengths, 5 mg, 10 mg and 20 mg of Vardenafil in the hydrochloride trihydrate form. The core of the tablet contains crospovidone, magnesium stearate, microcrystalline cellulose and colloidal anhydrous silicon dioxide as pharmaceutical excipients. The coating of the tablet contains macrogol 400, hypromellose, titanium dioxide, yellow iron trioxide, red iron trioxide and colloidal anhydrous silicon dioxide. The pharmaceutical formulation is produced by dry granulation followed by conventional processes used in preparation of solid dosage forms (EMA, Scientific discussion, Oct 21, 2005).

The following composition of the tablet core has been determined experimentally:

| Levitra (tablet core)-composition | 5 mg | | 10 mg | | 20 mg | |
|---|---|---|---|---|---|---|
| | weight% | mg/tbl | weight% | mg/tbl | weight% | mg/tbl |
| Vardenafil .HCl.3H₂0 | 6.2 | 5.93 | 9.1 | 11.92 | 12.7 | 23.62 |
| Crospovidone | 7.2 | 6.80 | 7.1 | 9.35 | 6.4 | 11.90 |
| Magnesium stearate | 0.6 | 0.57 | 0.8 | 1.02 | 0.9 | 1.65 |
| Microcrystalline cellulose | 85.6 | 81.30 | 81.9 | 107.28 | 79.7 | 148.28 |
| Colloidal anhydrous silicon oxide | 0.4 | 0.40 | 1.1 | 1.43 | 0.3 | 0.55 |
| Sum | 100.0 | 95.00 | 100.0 | 131.00 | 100.0 | 186.00 |

The international patent application WO2004/006894A says that Vardenafil hydrochloride occurs in four different polymorphous forms (anhydrous modification I with the melting temperature of 217°C, modification II with the melting temperature of 190°C, modification III with the melting temperature of 183-186°C, modification IV with the transformation temperature of 166°C), which can, depending on the ambient humidity and temperature, contain variable quantities of water and can form other pseudo-polymorphous forms with water. The application mentions that none of these polymorphous forms is preferentially formed at the room temperature, but for the preparation of a dosage form the active substance has to be used in a defined and reproducible form. This problem is solved by a method of production of the drug in the solid form, which starts from Vardenafil hydrochloride with an arbitrary content of water, which is carried out in the trihydrate form by being exposed to humidified gas at an intermediate stage or in the state of the finished product state until at least 90 mol % of Vardenafil hydrochloride trihydrate are formed.

During experimental testing in accelerated stability tests or in stress tests it has been found that Vardenafil hydrochloride trihydrate in the commercially available preparation Levitra® partly passes into Vardenafil base (20-30% by weight of the base have been found).

Inasmuch the base and a salt of one active substance usually exhibit different solubilities, which may be manifested in different biological availabilities, maximum plasma concentrations or the times of achieving the maximum plasmatic concentration, it is necessary that the active substance remains in an unchanged form in the pharmaceutical formulation throughout the whole usability period.

The present invention solves the above mentioned problem by providing a stable pharmaceutical composition containing Vardenafil hydrochloride, preferably Vardenafil hydrochloride trihydrate, stearic acid and at least one more pharmaceutically acceptable excipient. It has surprisingly been found out that the pharmaceutical formulation according to the invention, for the preparation of which Vardenafil hydrochloride was used in the trihydrate form, is stable and there does not occur formation of the Vardenafil base or change of the amount of water, i.e. transition of Vardenafil hydrochloride trihydrate into another hydrate or anhydrous form of Vardenafil hydrochloride, while the composition does not need to be contacted with humidified gas in any processing stage.

### Disclosure of Invention

The invention provides a stable pharmaceutical formulation containing Vardenafil hydrochloride, or, preferably, Vardenafil hydrochloride trihydrate, stearic acid and at least one pharmaceutically acceptable excipient.

The stable pharmaceutical formulation is suitable for oral administration, while it is in a solid form such as powder, granules, coated tablets, hard gelatine capsules, preferably in the form of a tablet or film coated tablet.

Suitable pharmaceutically acceptable excipients include fillers, disintegrants and optionally excipients. The pharmaceutical formulation contains microcrystalline cellulose as a filler, crospovidone as a disintegrant, and silicon dioxide as a further excipient. Stearic acid fulfils the role of a glidant in the formulation, preferably in an amount of 1 to 4% by weight.

The stable pharmaceutical formulation is prepared by granulation of Vardenafil hydrochloride, preferably Vardenafil hydrochloride trihydrate, stearic acid and at least one pharmaceutically acceptable excipient. A granulate is prepared form a mixture of Vardenafil hydrochloride, preferably Vardenafil hydrochloride trihydrate, stearic acid and at least one pharmaceutically acceptable excipient, which granulate is subsequently compressed into tablets and optionally provided with a coating. Preparation of the granulate by dry granulation by compaction is preferred, preferably with compression pressures of 5 to 7 MPa (50 to 70 bar).

In a preferable embodiment, 1/3 of the amount of stearic acid is added to the mixture for dry granulation, the rest being added to the granulate before compression into tablets. The tablet cores are optionally coated with a film at a temperature not exceeding 40°C.

Methods used for testing of stability of Vardenafil hydrochloride, preferably Vardenafil hydrochloride trihydrate, in a composition and in binary mixtures:
Stress test - 20 tablets/cores or ca. 5 g of the binary mixture were, in a Petri dish, placed into a climatic chamber and left at the temperature of 80 °C and relative humidity of 75% for up to 64 hours.
Accelerated stability test - was performed in a chamber at 40°C and 75% relative humidity.

### Brief Descrintion of Drawings

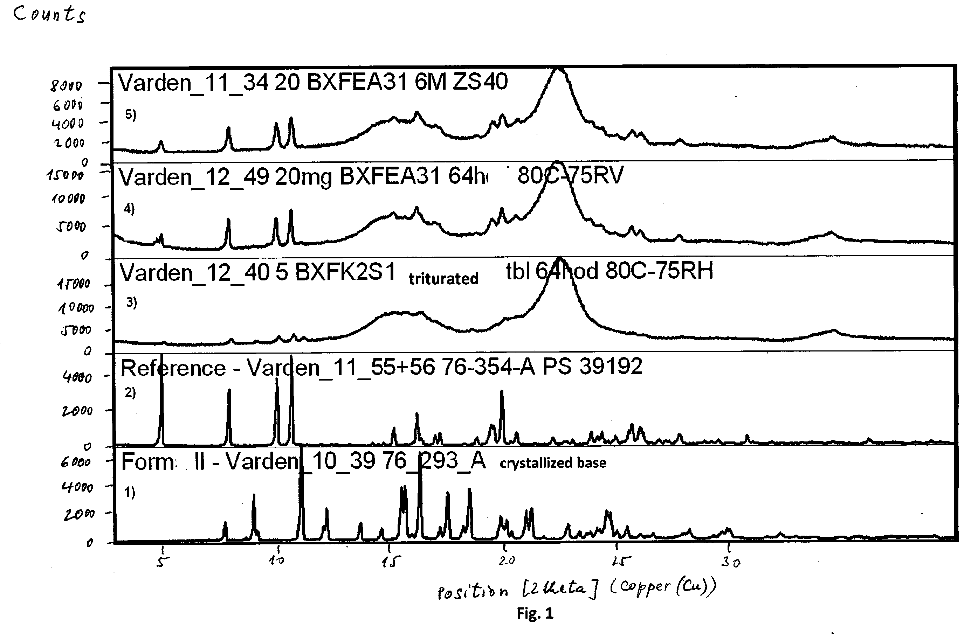

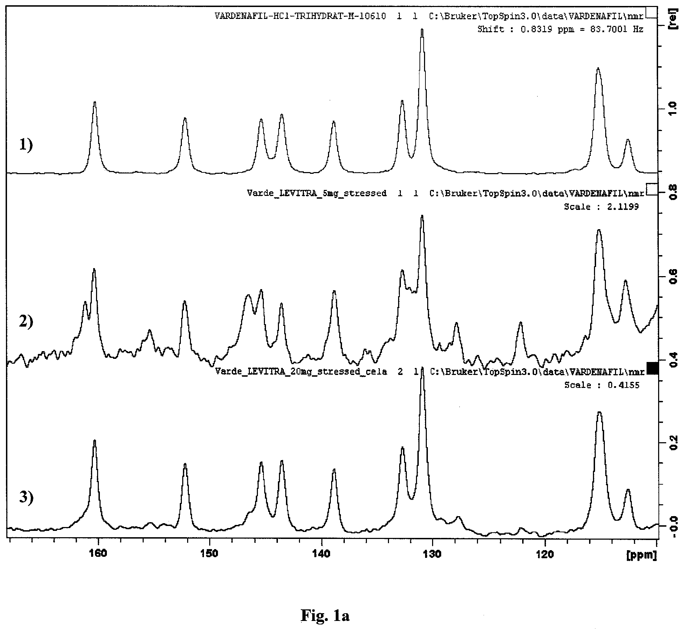

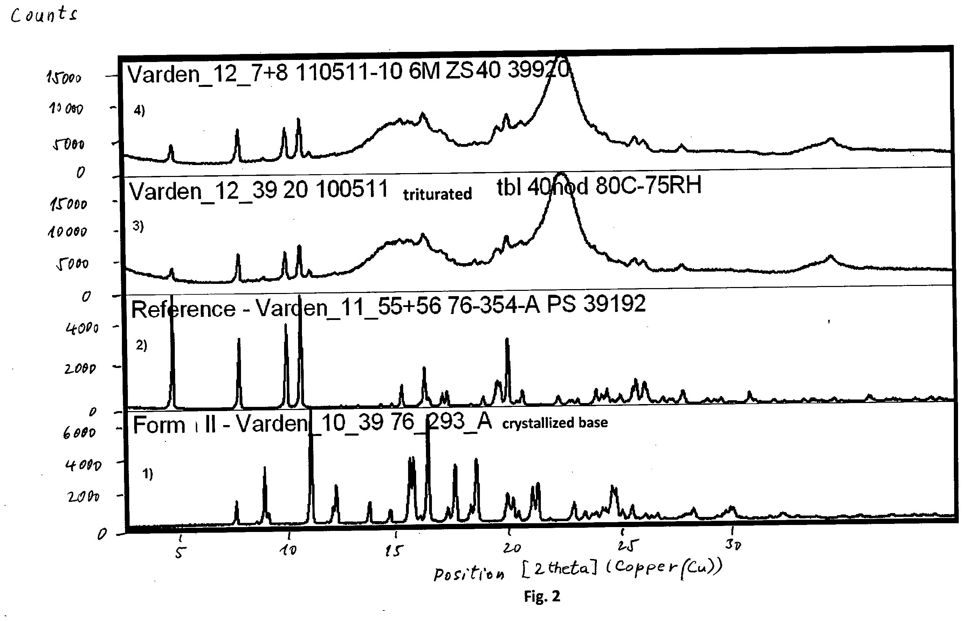

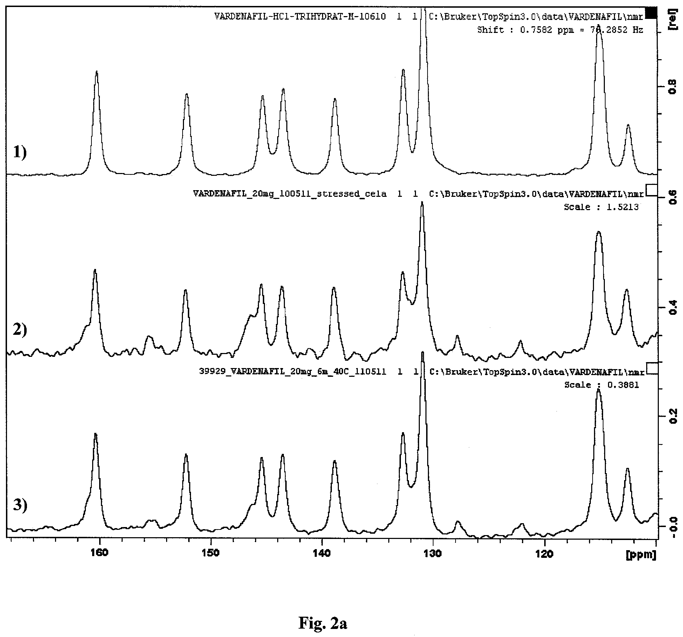

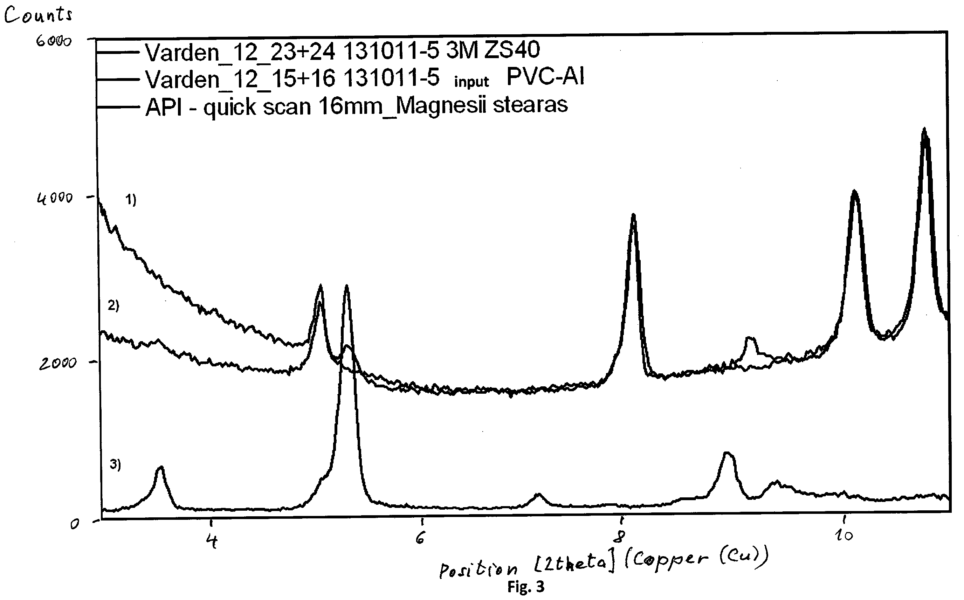

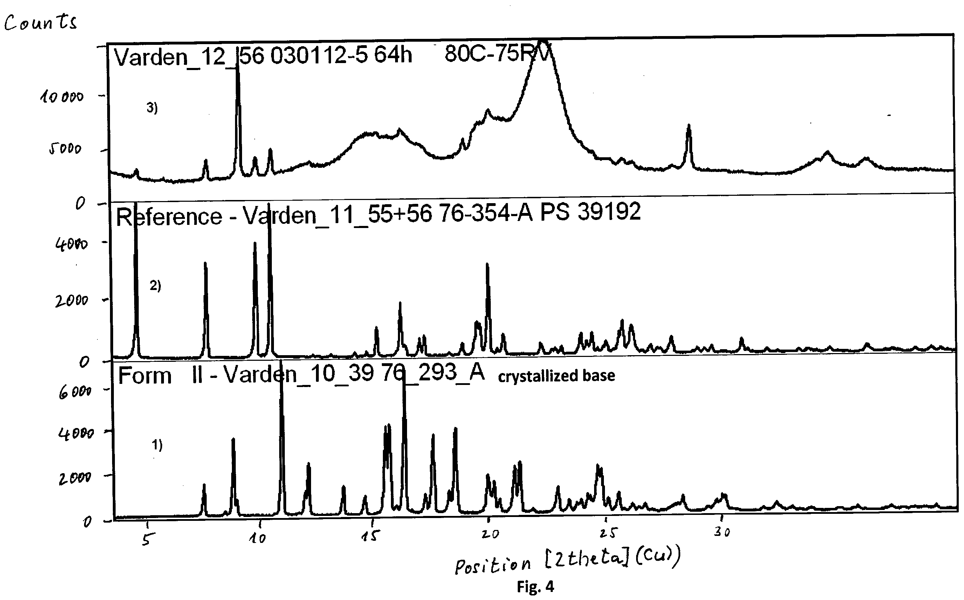

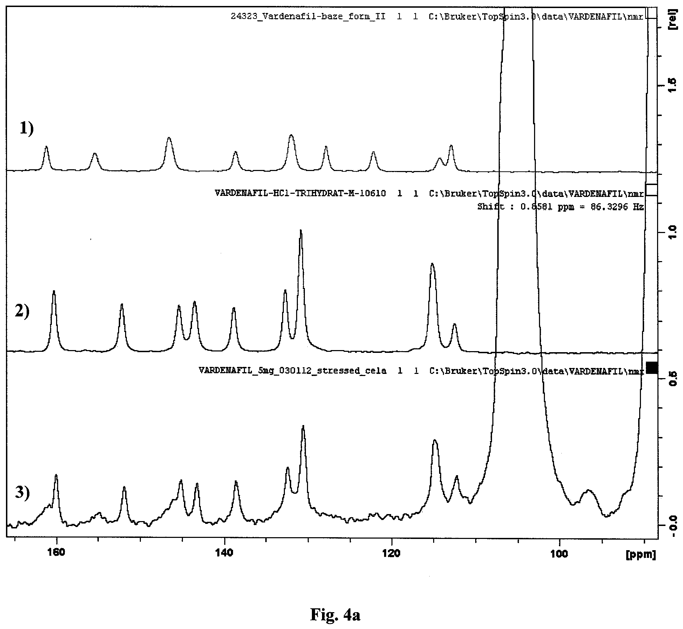

**Fig. 1****:** Formation of the base in the Levitra® preparation having the strengths of 20 mg and 5 mg in accelerated stability tests and in stress tests
   1) X-ray diffraction pattern of Vardenafil base
   2) X-ray diffraction pattern of Vardenafil hydrochloride trihydrate
   3) X-ray diffraction pattern of Levitra® 5 mg after a stress test (64 h, 80°C, 75% relative humidity)
   4) X-ray diffraction pattern of Levitra® 20 mg after a stress test (64 h, 80°C, 75% relative humidity)
   5) X-ray diffraction pattern of Levitra® 20 mg after a 6-month accelerated stability test
**Fig. 1a****:** NMR spectra of the Levitra® preparation 20 mg and 5 mg after a stress test
   1) 13C ss NMR spectrum of Vardenafil hydrochloride trihydrate
   2) 13C ss NMR spectrum of Levitra® 5 mg after a stress test (64 h, 80°C, 75% relative humidity)
   3) 13C ss NMR spectrum of Levitra® 20 mg after a stress test (64 h, 80°C, 75% relative humidity)
**Fig. 2****:** Formation of similar amounts of the Vardenafil base in a formulation having the strength of 20 mg, prepared in accordance with Example 1 (comparative example) after a stress test and accelerated stability tests. It can be seen from X-ray diffraction patterns 3) and 4) that the stress test simulates accelerated stabilities.
   1) X-ray diffraction pattern of Vardenafil base
   2) X-ray diffraction pattern of Vardenafil hydrochloride trihydrate
   3) X-ray diffraction pattern of Vardenafil 20 mg after a stress test (40 h, 80°C, 75% relative humidity)
   4) X-ray diffraction pattern of Vardenafil 20 mg after a 6-month accelerated stability test
**Fig. 2a****:** NMR spectra of a formulation having the 20 mg strength prepared in accordance with Example 1 (comparative example) after a stress test and an accelerated stability test
   1) 13C ss NMR spectrum of Vardenafil hydrochloride trihydrate
   2) 13C ss NMR spectrum of Vardenafil 20 mg after a stress test (40h, 80°C, 75 % relative humidity)
   3) 13C ss NMR spectrum of Vardenafil 20 mg after a 6-month accelerated stability test
**Fig. 3****:** Change of magnesium stearate in a formulation having the 5 mg strength prepared in accordance with Example 1. It appears that magnesium stearate is responsible for the formation of the base inasmuch its content decreases in the accelerated stability tests
   1) X-ray diffraction pattern of Vardenafil 5 mg after a 3-month accelerated stability test
   2) X-ray diffraction pattern of Vardenafil 5 mg without loading
   3) X-ray diffraction pattern of magnesium stearate
**Fig. 4****:** Stress test of a formulation having the 5 mg strength prepared in accordance with Example 3 (magnesium stearate replaced with talc) during which no base forms
   1) X-ray diffraction pattern of Vardenafil base
   2) X-ray diffraction pattern of Vardenafil hydrochloride trihydrate
   3) X-ray diffraction pattern of Vardenafil 5 mg prepared in accordance with Example 3 after a stress test (64 h, 80°C, 75% relative humidity)
**Fig. 4a****:** NMR spectra of a formulation having the 5 mg strength prepared in accordance with Example 3 (magnesium stearate is replaced with talc) after a stress test
   1) 13C ss NMR spectrum of Vardenafil base
   2) 13C ss NMR spectrum of Vardenafil hydrochloride trihydrate
   3) 13C ss NMR spectrum of Vardenafil 5 mg prepared in accordance with Example 3 after a stress test (64 h, 80°C, 75% relative humidity)

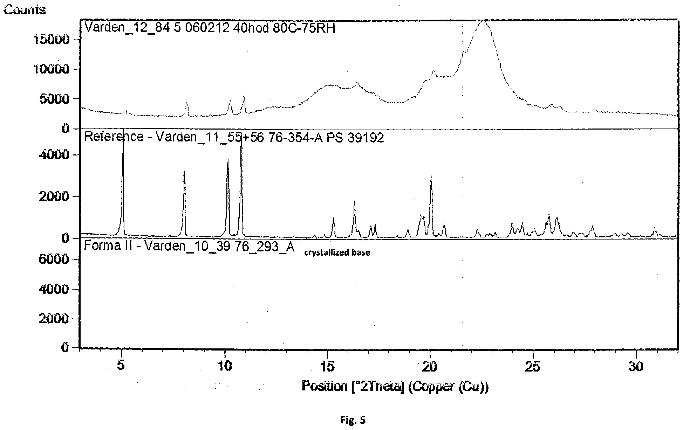

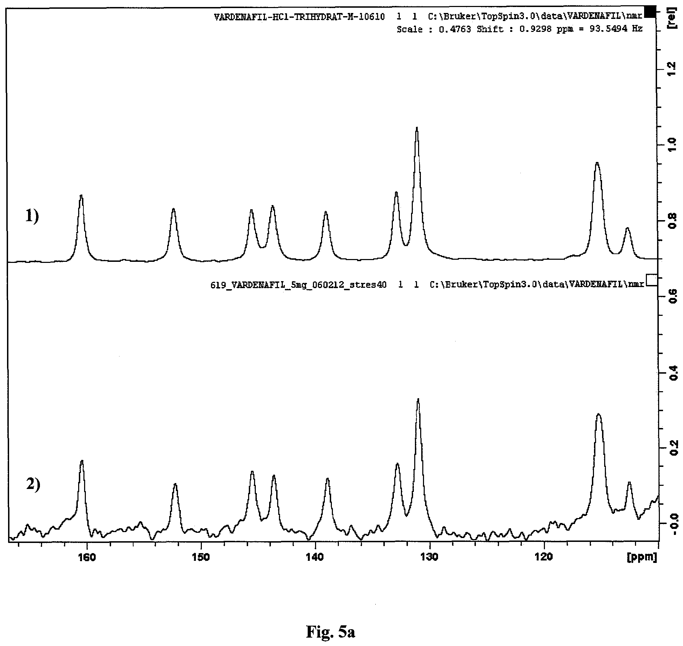

**Fig. 5****:** Stress test of a formulation having the 5 mg strength prepared in accordance with Example 5 C (magnesium stearate is replaced with stearic acid) in which no base forms
   1) X-ray diffraction pattern of Vardenafil base
   2) X-ray diffraction pattern of Vardenafil hydrochloride trihydrate
   3) X-ray diffraction pattern of Vardenafil 5 mg prepared in accordance with Example 5 C after a stress test (40 h, 80°C, 75% relative humidity)
**Fig. 5a****:** NMR spectra after a stress test of a formulation having the 5 mg strength prepared in accordance with Example 5 C (magnesium stearate is replaced with stearic acid)
   1) 13C ss NMR spectrum of Vardenafil hydrochloride trihydrate
   2) 13C ss NMR spectrum of Vardenafil 5 mg prepared in accordance with Example 5 C, stress test (40 h, 80°C, 75% relative humidity)

### Examples

### Example 1. Comparative example

| Zentiva | | 5 mg | | 20 mg | |
|---|---|---|---|---|---|
| | | weight % | mg/tbl | weight % | mg/tbl |
| Vardenafil | 488.604 g/mol | | 5.000 | | 20.000 |
| Vardenafil.HCl.3H₂0 | 579.1 g/mol | 5.926 | 5.926 | 11.852 | 23.704 |
| Avicel 101 | | 87.574 | 87.574 | 81.648 | 163.296 |
| SiO₂ | | 0.500 | 0.500 | 0.500 | 1.000 |
| Polyplasdone XL | | 5.000 | 5.000 | 5.000 | 10.000 |
| Magnesium stearate | | 1.000 | 1.000 | 1.000 | 2.000 |
| Table core | | | 100.000 | | 200.000 |
| Methocel (SAP-1862) | | | 1.5313 | | 3.035 |
| PEG 400 | | | 0.2940 | | 0.583 |
| TiO₂ | | | 0.1715 | | 0.340 |
| Fe₂O₃ - yellow | | | 0.0031 | | 0.039 |
| Fe₂O₃ - red | | | 0.0001 | | 0.003 |
| Tablet coating | | | 2.000 | | 4.000 |
| Sum | | | 102.000 | | 204.000 |

A dosage form was prepared using a dry granulation method by compaction with subsequent compression in a rotary tabletting machine and coating. The raw materials Vardenafil hydrochloride trihydrate, microcrystalline cellulose, polyplasdone, silicon dioxide and magnesium stearate were screened through a sieve with the mesh size of 0.8 mm.
A mixture of Vardenafil hydrochloride trihydrate, microcrystalline cellulose, polyplasdone and silicon dioxide was homogenized at 30 rpm for 10 min. A half of the total amount of, or, alternatively, no magnesium stearate was added to the mixture and the mixture was homogenized at 30 rpm for 5 min. After subsequent dry granulation carried out in a Powtec compactor with the compression pressure of 50-70 bar the resulting granulate was screened through a sieve with the mesh size of 0.8 mm. The remaining amount of magnesium stearate was admixed to the screened granulate and the mixture was homogenized at 30 rpm for 5 min. Thus prepared tabletting blend was compressed in a rotary tabletting machine. The 20 mg strength was compressed to lens-shaped tablets with the diameter of 8 mm, 5 mg strength was compressed to lens-shaped tablets with the diameter of 6 mm. The cores were coated with Methocel suspension (cf. the table of Example 1 for composition), wherein the temperature inside the coating drum did not exceed 40°C during the spraying.

A portion of the resulting tablets was left at a temperature of 20-25 °C and relative air humidity of 40 - 80% for at least 24 hours before filling into the primary package.

A stress test, during which 20 tablets/cores prepared in the above mentioned way were inserted in a climatic chamber in a Petri dish and left at the temperature of 80°C and relative humidity of 75% for up to 64 hours, proved formation of Vardenafil base both in tablets exposed to humid air for at least 24 hours and in tablets filled in the primary package immediately after the preparation. Approximately 30% by weight of Vardenafil hydrochloride trihydrate passed into Vardenafil base in the case of 5 mg strength and approximately 25% by weight in the case of 20 mg strength. The effect of transformation of Vardenafil hydrochloride trihydrate to Vardenafil base in the composition was more pronounced in the 5 mg strength as opposed to the 20 mg strength, for the reason of non-proportionality of the composition; the 5 mg strength exhibits a higher stoichiometric ratio of magnesium stearate/Vardenafil hydrochloride trihydrate. In addition, a phase shift of magnesium stearate was observed, which led to the assumption that basic magnesium stearate was responsible for transformation of Vardenafil hydrochloride trihydrate to Vardenafil base.

It has further been verified that it has no influence on formation of Vardenafil base in the composition whether all the mixture of the final composition is compacted, or whether possibly 50-100% of the lubricant is only admixed after the compaction.

### Example 2. Binary mixtures

| | Formation of base |
|---|---|
| Vardenafil HCl.3H₂O | No |
| Magnesium stearate | - |
| Vardenafil HCl.3H₂O + Magnesium stearate (1:1 m/m) | Yes |
| Vardenafil HCl.3H₂O + Talc (1:1 m/m) | No |
| Vardenafil HCl.3H₂O + Sodium stearyl fumarate (1:1 m/m) | No |
| Vardenafil HCl.3H₂O + stearic acid (1:1 m/m) | No |

Mixture of vardenafil hydrochloride trihydrate and stearic acid is according to the invention.

Binary mixtures were prepared in accordance with the table and subjected to stress tests during which approximately 5 g of the primary mixture in a Petri dish was inserted in the climatic chamber and left at the temperature of 80°C and relative humidity of 75% for 64 hours. The stress tests of compatibility in binary mixtures have confirmed that the cause of transformation of Vardenafil hydrochloride trihydrate to Vardenafil base was magnesium stearate.

### Example 3. Composition containing talc(reference example)

A composition containing the ingredients as shown in the table below was prepared using the procedure described in Example 1; however, magnesium stearate was replaced with talc.

| | | 5 mg | |
|---|---|---|---|
| | | weight % | mg/tbl |
| Vardenafil | 488.604 g/mol | | 5.000 |
| Vardenafil.HCl.3H₂0 | 579.1 g/mol | 5.926 | 5.926 |
| Avicel 101 | | 83.574 | 83.574 |
| SiO₂ | | 0.500 | 0.500 |
| Polyplasdone XL | | 5.000 | 5.000 |
| Talc | | 5.000 | 5.000 |
| Table core | | | 100.000 |
| Methocel (SAP-1862) | | | 1.5313 |
| PEG 400 | | | 0.2940 |
| TiO₂ | | | 0.1715 |
| Fe₂O₃ - yellow | | | 0.0031 |
| Fe₂O₃ - red | | | 0.0001 |
| Tablet coating | | | 2.000 |
| Sum | | | 102.000 |

A stress test during which 20 tablets/cores prepared in the above mentioned way were inserted in a climatic chamber in a Petri dish and left at the temperature of 80°C and relative humidity of 75% for up to 64 hours has confirmed that no transformation of Vardenafil hydrochloride trihydrate to Vardenafil base occurred, i.e. a direct influence of the other excipients of the composition on the formation of base was excluded.

### Example 4. Composition containing sodium stearyl fumarate (reference example)

A composition containing the ingredients as shown in the table below was prepared using the procedure described in Example 1; however, magnesium stearate was replaced with sodium stearyl fumarate.

| Zentiva | | 5 mg | |
|---|---|---|---|
| | | weight % | mg/tbl |
| Vardenafil | 488.604 g/mol | | 5.000 |
| Vardenafil.HCl.3H₂0 | 579.1 g/mol | 5.926 | 5.926 |
| Avicel 101 | | 87.574 | 87.574 |
| SiO₂ | | 0.500 | 0.500 |
| Polyplasdone XL | | 5.000 | 5.000 |
| Sodium stearyl fumarate | | 1.000 | 1.000 |
| Tablet core | | | 100.000 |
| Methocel (SAP-1862) | | | 1.5313 |
| PEG 400 | | | 0.2940 |
| TiO₂ | | | 0.1715 |
| Fe₂O₃ - yellow | | | 0.0031 |
| Fe₂O₃ - red | | | 0.0001 |
| Tablet coating | | | 2.000 |
| Sum | | | 102.000 |

A stress test during which 20 tablets/cores prepared in the above mentioned way were inserted in a climatic chamber in a Petri dish and left at the temperature of 80°C and relative humidity of 75% for up to 64 hours has confirmed that no transformation of Vardenafil hydrochloride trihydrate to Vardenafil base occurred.

### Example 5. Compositions containing stearic acid

The following compositions were prepared:

| Exp No | Vardenafil hydrochloride trihydrate [weight %] | Polyplasdone XL [weight %] | Aerosil [weight %] | Stearic acid [weight %] | Avicel [weight %] | Avicel type | Vardenafil base |
|---|---|---|---|---|---|---|---|
| A | 5.93 | 5.00 | 0.50 | 1.00 | 87.57 | PH101 | no |
| B | 5.93 | 5.00 | 0.50 | 1.00 | 87.57 | PH112 | no |
| C | 5.93 | 5.00 | 0.50 | 4.00 | 84.57 | PH101 | no |
| D | 5.93 | 5.00 | 0.50 | 4.00 | 84.57 | PH112 | no |
| E | 5.93 | 5.00 | 0.50 | 2.50 | 86.07 | PH101 | no |
| F | 5.93 | 5.00 | 0.50 | 2.50 | 86.07 | PH101 | no |
| G | 5.93 | 5.00 | 0.50 | 2.50 | 86.07 | PH101 | no |

| | | | | | | | |
|---|---|---|---|---|---|---|---|
| Polyplasdone XL - crosslinked homopolymers of N-vinyl-2-pyrrolidone Aerosil - colloidal anhydrous silicon dioxide Avicel - microcrystalline cellulose, PH101 - nominal mean particle size 50 µm, water content ≤ 5.0 %; PH112 - nominal mean particle size 100 µm, water content ≤ 1.5 % | | | | | | | |

The raw materials Vardenafil hydrochloride trihydrate, microcrystalline cellulose, polyplasdone, silicon dioxide and stearic acid were screened through a sieve with the mesh size of 0.8 mm. The homogenization was carried out in 2 steps in a Turbula device. A mixture of Vardenafil hydrochloride trihydrate, microcrystalline cellulose, polyplasdone, silicon dioxide, stearic acid (1/3 of the total amount) was homogenized at 30 rpm for 10 min. The subsequent dry granulation was carried out in a Powtec compactor with the compression pressure of 5 - 7 MPa (50-70 bar) and the resulting granulate was screened through a sieve with the mesh size of 0.8 mm. The remaining amount of stearic acid was admixed to the screened granulate and the mixture was homogenized at 30 rpm for 5 min. Thus prepared tabletting blend was compressed in a rotary tabletting machine to lens-shaped tablets with the diameter of 6 mm. The cores were coated with Methocel suspension (composition identical to the coating of Example 1), wherein the temperature inside the coating drum did not exceed 40°C during the spraying.

A stress test during which 20 cores in a Petri dish were inserted in a climatic chamber and left at the temperature of 80°C and relative humidity of 75% for 40 hours proved that none of the composition exhibited transformation of Vardenafil hydrochloride trihydrate to Vardenafil base after a stress test.

## Claims

1. A stable pharmaceutical formulation, **characterized in that** it contains an effective amount of Vardenafil hydrochloride or Vardenafil hydrochloride trihydrate, stearic acid and at least one further pharmaceutically acceptable excipient.

2. The stable pharmaceutical formulation according to claim 1, **characterized in that** it contains Vardenafil hydrochloride trihydrate.

3. The stable pharmaceutical formulation according to claim 1, **characterized in that** it contains 1 to 4% by weight of stearic acid.

4. The stable pharmaceutical formulation according to claim 1, **characterized in that** said further pharmaceutically acceptable excipient is microcrystalline cellulose, crospovidone, silicon dioxide or a mixture thereof.

5. The stable pharmaceutical formulation according to any one of the preceding claims, **characterized in that** it is in an oral dosage form (for oral administration).

6. The stable pharmaceutical formulation according to any one of the preceding claims, **characterized in that** it is in the form of a tablet or film coated tablet.

7. A process for the production of the stable pharmaceutical formulation as defined in claims 1 to 6, **characterized in that** a granulate is prepared from a mixture of Vardenafil hydrochloride or Vardenafil hydrochloride trihydrate, stearic acid and at least one pharmaceutically acceptable excipient, which granulate is subsequently compressed to tablets, and optionally provided with a coating in the form of a film.

8. The process according to claim 7, **characterized in that** 1/3 of the amount of the total amount of stearic acid is added to the mixture before granulation and the remaining amount is added to the granulate before compression to tablets.

9. The process according to claim 8, **characterized in that** the granulate is prepared by dry granulation, preferably compaction.

10. The process according to claim 9, **characterized in that** the compaction is carried out with the compression pressure of 5 to 7 MPa.

11. The process according to claim 7, **characterized in that** the compressed tablet is provided with a film coating at a temperature not exceeding 40°C.

## Patentansprüche

1. Stabile pharmazeutische Formulierung, **dadurch gekennzeichnet, dass** sie eine wirksame Menge von Vardenafilhydrochlorid oder Vardenafilhydrochloridtrihydrat, Stearinsäure und mindestens einen weiteren pharmazeutisch annehmbaren Hilfsstoff enthält.

2. Stabile pharmazeutische Formulierung nach Anspruch 1, **dadurch gekennzeichnet, dass** sie Vardenafilhydrochloridtrihydrat enthält.

3. Stabile pharmazeutische Formulierung nach Anspruch 1, **dadurch gekennzeichnet, dass** sie 1 bis 4 Gew.-% Stearinsäure enthält.

4. Stabile pharmazeutische Formulierung nach Anspruch 1, **dadurch gekennzeichnet, dass** der weitere pharmazeutisch annehmbare Hilfsstoff mikrokristalline Cellulose, Crospovidon, Siliciumdioxid oder eine Mischung davon ist.

5. Stabile pharmazeutische Formulierung nach einem der vorhergehenden Ansprüche, **dadurch gekennzeichnet, dass** sie als orale Dosierungsform (zur oralen Verabreichung) vorliegt.

6. Stabile pharmazeutische Formulierung nach einem der vorhergehenden Ansprüche, **dadurch gekennzeichnet, dass** sie in Form einer Tablette oder Filmtablette vorliegt.

7. Verfahren zur Herstellung der stabilen pharmazeutischen Formulierung nach einem der Ansprüche 1 bis 6, **dadurch gekennzeichnet, dass** aus einem Gemisch aus Vardenafilhydrochlorid oder Vardenafilhydrochloridtrihydrat, Stearinsäure und mindestens einem pharmazeutisch annehmbaren Hilfsstoff ein Granulat hergestellt wird, wobei das Granulat anschließend zu Tabletten verpresst und wahlweise mit einem Überzug in Form eines Films versehen wird.

8. Verfahren nach Anspruch 7, **dadurch gekennzeichnet, dass** dem Gemisch vor der Granulierung 1/3 der Menge der Gesamtmenge an Stearinsäure zugesetzt wird und die verbleibende Menge dem Granulat vor dem Verpressen zu Tabletten zugesetzt wird.

9. Verfahren nach Anspruch 8, **dadurch gekennzeichnet, dass** das Granulat durch Trockengranulierung, vorzugsweise Kompaktierung, hergestellt wird.

10. Verfahren nach Anspruch 9, **dadurch gekennzeichnet, dass** die Kompaktierung unter Anwendung eines Verpressungsdrucks von 5 bis 7 MPa durchgeführt wird.

11. Verfahren nach Anspruch 7, **dadurch gekennzeichnet, dass** die gepresste Tablette bei einer Temperatur von nicht über 40 °C mit einem Filmüberzug versehen wird.

## Revendications

1. Une formulation pharmaceutique stable, **caractérisée en ce qu'**elle contient une quantité efficace de chlorhydrate de Vardenafil ou de chlorhydrate de Vardenafil trihydraté, d'acide stéarique et d'au moins un excipient pharmaceutiquement acceptable supplémentaire.

2. La formulation pharmaceutique stable selon la revendication 1, **caractérisée en ce qu'**elle contient du chlorhydrate de Vardenafil trihydraté.

3. La formulation pharmaceutique stable selon la revendication 1, **caractérisée en ce qu'**elle contient de 1 à 4% en poids d'acide stéarique.

4. La formulation pharmaceutique stable selon la revendication 1, **caractérisée en ce que** ledit excipient pharmaceutiquement acceptable supplémentaire est la cellulose microcristalline, la crospovidone, le dioxyde de silicium ou un mélange de ceux-ci.

5. La formulation pharmaceutique stable selon l'une quelconque des revendications précédentes, **caractérisée en ce qu'**elle est sous une forme galénique orale (pour administration orale).

6. La formulation pharmaceutique stable selon l'une quelconque des revendications précédentes, **caractérisée en ce qu'**elle se présente sous la forme d'un comprimé ou d'un comprimé revêtu d'un pelliculage.

7. Un procédé de production de la formulation pharmaceutique stable telle que définie dans les revendications 1 à 6, **caractérisé en ce que** l'on prépare des granules à partir d'un mélange de chlorhydrate de Vardenafil ou chlorhydrate de Vardenafil trihydraté, d'acide stéarique et d'au moins un excipient pharmaceutiquement acceptable, lesquels granules sont par la suite compressés en comprimés, et éventuellement dotés d'un revêtement sous forme d'un pelliculage.

8. Le procédé selon la revendication 7, **caractérisé en ce que** 1/3 de la quantité totale de l'acide stéarique est ajouté au mélange avant la granulation et la quantité restante est ajoutée aux granules avant leur compression en comprimés.

9. Le procédé selon la revendication 8, **caractérisé en ce que** le granulat est préparé par granulation à sec, de préférence par compactage.

10. Le procédé selon la revendication 9, **caractérisé en ce que** le compactage est effectué sous une pression de compression de 5 à 7 MPa.

11. Le procédé selon la revendication 7, **caractérisé en ce que** le comprimé compressé est pourvu d'un pelliculage à une température ne dépassant pas 40°C.
